# EUROPEAN PATENT APPLICATION

(11) **EP 4 542 194 A1**
(43) Date of publication of application: **23.04.2025**
(21) Application number: 23823888.5
(22) Date of filing: 12.06.2023
(51) Int. Cl.: G01N 1/22, G01N 1/00, G01N 30/04

(54) **GAS CONTINUOUS ANALYSIS SYSTEM, AND GAS CONTINUOUS ANALYSIS METHOD**

(30) Priority: 16.06.2022 JP 2022097368; 17.08.2022 JP 2022130052; 22.12.2022 JP 2022205211
(71) Applicant: HORIBA, Ltd., Kyoto-shi Kyoto 601-8510 (JP)
(72) Inventor: TACHIBANA, Kohei, Kyoto-shi, Kyoto 601-8510 (JP); IDO, Takuya, Kyoto-shi, Kyoto 601-8510 (JP); ISHIZAKI, Yukihiro, Kyoto-shi, Kyoto 601-8510 (JP); TSUKATANI, Kosuke, Kyoto-shi, Kyoto 601-8510 (JP); NAKAMURA, Keishi, Kyoto-shi, Kyoto 601-8510 (JP)
(74) Representative: Müller Hoffmann & Partner
(86) International application number: PCT/JP2023/021728
(87) International publication number: WO 2023/243598

(57) **Abstract**

This invention is to shorten time to calibrate or validate an analyzer that analyzes a sample gas containing an adsorbable component, and comprises a main flow channel through which the sample gas containing the adsorbable component flows, an analyzer that analyzes the adsorbable component in the sample gas flowing in the main flow channel, a standard gas flow channel that supplies a standard gas for calibration or validation to the analyzer, and a purge gas flow channel that supplies a purge gas containing moisture to the analyzer, and continuously analyzes the sample gas containing moisture by the analyzer by introducing the sample gas into the main flow channel, purges the analyzer by supplying the purge gas to the analyzer from the purge gas flow channel in a middle of continuous analysis and successively calibrates or validates the analyzer by supplying the standard gas to the analyzer from the standard gas flow channel.

## Description

### Technical field

This invention relates to a gas continuous analysis system and a gas continuous analysis method.

### Background Art

Conventionally, as a method for continuous analysis of a gas (for example, a flue gas, a process gas, or the like) emitted from, for example, industrial facilities such as industrial waste treatment plants and chemical plants, a gas is sampled and the sampled gas is introduced into an analyzer. The analyzer is then calibrated (zero calibration, span calibration) during a course of this continuous analysis. The zero calibration is performed using a zero gas as nitrogen (N₂) gas, and the span calibration is performed using a span gas containing a measured component of a known concentration.

However, in case of analyzing an adsorbable component such as hydrogen fluoride (HF) or hydrogen chloride (HCl) contained in the gas, the adsorbable component is adsorbed on an inner surface of a piping or a measurement cell of the analyzer, and in case that the zero gas is flown during calibration, the adsorbable component adsorbed in the measurement cell is separated from the measuring cell. Then, it takes time for the indicated value of the analyzer at the time of calibration to stabilize. As a result of this, the calibration time between consecutive analyses becomes longer, and the interruption time in gas continuous analysis becomes longer.

### Prior art documents

### Patent document

Patent Document 1: Japan Unexamined Patent Application Publication No. 2013-96889

### Summary of the Invention

### Problems to be solved by the invention

The present invention is to solve the above-mentioned problem and a main object of this invention is to shorten time for calibration or validation of an analyzer that analyzes a sample gas containing an adsorbable component.

### Means to solve the problem

More specifically, a gas continuous analysis system in accordance with this invention comprises a main flow channel through which a sample gas containing an adsorbable component flows, an analyzer that analyzes the adsorbable component in the sample gas flowing in the main flow channel, a standard gas flow channel that supplies a standard gas for calibrating or validating the analyzer, and a purge gas flow channel that supplies a purge gas containing moisture to a channel through which the sample gas of the analyzer passes, and is characterized by that the sample gas is introduced into the main flow channel and the sample gas is continuously analyzed by the analyzer, and in the middle of the continuous analysis, the purge gas is supplied to the analyzer from the purge gas flow channel to purge the flow channel through which the sample gas of the analyzer passes, and successively a standard gas is supplied to the analyzer from the standard gas flow channel to calibrate or validate the analyzer.

In accordance with the gas continuous analysis system, since the flow channel in which the sample gas of the analyzer flows is purged by using the purge gas containing moisture prior to calibration or validation of the analyzer, it is possible to shorten the time required for stabilizing the indicated value of the analyzer during calibration or validation. Since the purge gas containing moisture is used, the adsorbable component, which is adsorbed inside of the flow channel in which the sample gas of, for example, the measurement cell of the analyzer flows, is easily separated due to water molecules. As a result of this, it is possible to shorten the time for calibration or validation during an interval between continuous analyses, resulting in being able to shorten an interruption time of the continuous analysis of the sample gas.

As a concrete embodiment of the purge gas flow channel conceived is that the purge gas flow channel supplies atmosphere to the flow channel through which the sample gas of the analyzer gas passes. In accordance with this configuration, it is possible to reduce a cost for the purge gas.

As a concrete embodiment of the analyzer conceived is that the analyzer analyzes HF, HCl or NH₃ as the adsorbable component. Since a dipole moment as an index of adsorptive ability is H₂O>HF>NH₃>HCl, it is possible for the system that analyzes HF, HCL or NH₃ as the adsorbable component to make the effect of using the purge gas containing moisture remarkable

It is conceivable that the standard gas flow channel has a zero gas flow channel that supplies a zero gas to the analyzer, and a span gas flow channel that supplies a span gas to the analyzer.

With this configuration, in order to shorten an amount of time for zero calibration and an amount of time for span calibration, it is preferable that the gas continuous analysis system supplies the purge gas to the analyzer from the purge gas flow channel in the middle of continuous analysis to purge the flow channel through which the sample gas of the analyzer passes, successively supplies the zero gas from the zero gas flow channel to the analyzer to zero-calibrate the analyzer, and supplies the span gas to the analyzer from the span gas flow channel to span-calibrate the analyzer.

The standard gas flow channel may not have a zero gas flow channel that supplies the zero gas to the analyzer. In this case, it is preferable that the standard gas flow channel supplies the span gas to the analyzer, and the gas continuous analysis system supplies the purge gas to the analyzer from the purge gas flow channel to purge the flow channel through which the sample gas of the analyzer passes and to zero-calibrate the analyzer, and successively supplies the span gas to the analyzer from the standard gas flow channel to span-calibrate the analyzer.

It is possible to purge the downstream side of the connection point, of the purge gas flow channel to the main flow channel, in the main flow channel, however, it is not possible to purge the upstream side of the connection point in the main flow channel. Then, the exhaust gas stays after completion of the continuous analysis and the moisture in the exhaust gas condensates. This becomes a factor causing a measurement error in a subsequent continuous analysis.

In order to preferably solve this problem, it is preferable that the gas continuous analysis system further comprises a second purge gas flow channel that is connected to an upstream side of the analyzer in the main flow channel and that supplies a second purge gas from a connection point of the second purge gas flow channel to the main flow channel toward the upstream side of the main flow channel, and after the continuous analysis is completed, the gas continuous analysis system supplies the second purge gas from the second purge gas flow channel to purge the upstream side of the connection point in the second purge gas flow channel.

In case of analyzing an adsorbable component such as HF, HCL or NH₃, the adsorbable component adsorbs to an inner surface of the piping constituting the main flow channel so that a response time of the analyzer becomes longer.

In order to preferably solve this problem, it is preferable to further comprise an overflow flow channel that discharges a part of the sample gas flowing in the main flow channel in the upstream side of the analyzer.

In accordance with this configuration, it is possible to make the flow rate of the sample gas introduced into the main flow channel large by means of the overflow flow channel. As a result of this, it becomes possible to make the absorbable component difficult to absorb to the inner surface of the piping constituting the main flow channel.

As a configuration to shorten a distance from the overflow flow channel to the analyzer, it is preferable that a throttle unit is provided between a connection point of the overflow flow channel to the main flow channel and the analyzer in the main flow channel, a main suction pump is provided in the downstream side of the analyzer in the main flow channel, and a sub-suction pump is provided in the overflow flow channel.

In addition, the gas continuous analysis system in accordance with this invention is characterized by comprising a sampling unit that samples a gas containing an adsorbable component flowing in a duct, a main flow channel through which a sample gas sampled by the sampling unit flows, a chromatograph or a mass spectrometer that analyzes an adsorbable component in the sample gas flowing in the main flow channel, and an overflow channel that discharges a part of the sample gas flowing in the main flow channel in an upstream side of the chromatograph or the mass spectrometer.

In accordance with this configuration, it is possible to make the flow rate of the sample gas introduced into the main flow channel large by means of the overflow flow channel. As a result of this, it becomes possible to make the absorbable component difficult to absorb to the inner surface of the piping constituting the main flow channel, and to improve an analysis accuracy by the chromatograph or the mass spectrometer. In addition, it leads to improvement in the response time of the chromatograph or the mass spectrometer.

Furthermore, the gas continuous analysis method in accordance with this invention is a gas continuous analysis method using a measurement system comprising a main flow channel through which a sample gas containing an adsorbable component flows, an analyzer that analyzes the adsorbable component in the sample gas flowing in the main flow channel, a standard gas flow channel that supplies a standard gas for calibration or validation to the analyzer, and a purge gas flow channel that supplies a purge gas containing moisture to the flow channel through which the sample gas of the analyzer flows, and is characterized by that the sample gas is introduced into the main flow channel and the sample gas is continuously analyzed by the analyzer, the purge gas is supplied to the analyzer from the purge gas flow channel in the middle of continuous analysis to purge the flow channel through which the sample gas of the analyzer passes, and successively the standard gas is supplied to the analyzer from the standard gas flow channel to calibrate or validate the analyzer.

### Effects of the Invention

In accordance with the present claimed invention having the above-mentioned configuration, it is possible to shorten time required for calibration or validation of analyzers that analyze a sample gas containing an adsorbable component.

### Brief description of the drawings

[Fig. 1] A schematic diagram indicating a gas continuous analysis system in accordance with one embodiment of the present claimed invention.
[Fig. 2] A schematic diagram indicating a configuration of an inside of a housing that houses the analyzer of this embodiment.
[Fig. 3] A schematic diagram indicating a flow of a sample gas at a time of continuous analysis in this embodiment.
[Fig. 4] A schematic diagram indicating a flow of a purge gas (atmosphere) during a purge process in this embodiment.
[Fig. 5] A schematic diagram indicating a flow of a zero gas during zero calibration in this embodiment.
[Fig. 6] A schematic diagram indicating a flow of a span gas during span calibration in this embodiment.
[Fig. 7] A schematic diagram indicating a flow of a second purge gas after the end of continuous analysis (before or at the time of system shutdown) in this embodiment.
[Fig. 8] A schematic diagram indicating the gas continuous analysis system in accordance with a modified embodiment.
[Fig. 9] A schematic diagram indicating the gas continuous analysis system in accordance with a modified embodiment.

### Best mode for embodying the invention

One embodiment of a continuous analysis system in accordance with the present claimed invention will be described below with reference to drawings.

All figures shown below are schematically depicted with omissions or exaggerations for the sake of clarity. Identical components are indicated with the same codes and explanations are omitted as appropriate.

This gas continuous analysis system 100 in accordance with this embodiment continuously analyzes a concentration of an adsorbable component such as hydrogen fluoride (HF), hydrogen chloride (HCl) or ammonia (NH₃) in a sample gas.

In this embodiment, the sample gas is a combustion gas such as a gas under combustion or a combustion exhaust gas (a flue gas) or a process gas. The gas under combustion is a gas being burned in an external combustion engine, an industrial furnace, an incinerator, a turbine or a power plant or the like, and the flue gas is a post-combustion gas discharged from the external combustion engine, the industrial furnace, the incinerators, the turbine or the power plant. In addition, the process gas is a gas in a chemical plant such as petrochemistry, coal chemistry, natural gas chemistry, petroleum refining, methanation and gasifiers, and includes a raw material gas such as a natural gas as well as a gas separated or produced in a chemical plant. Furthermore, the process gas includes a gas that has passed through exhaust gas treatment facilities such as a scrubber in a semiconductor manufacturing plant (semiconductor manufacturing equipment), for example, an exhaust gas from etching equipment, which is passed through a scrubber that removes HF or the like generated in a decomposition reaction from the exhaust gas, and the exhaust gas after passing through the scrubber. In addition, the gas after detoxifying the above various gases may be used as the sample gas.

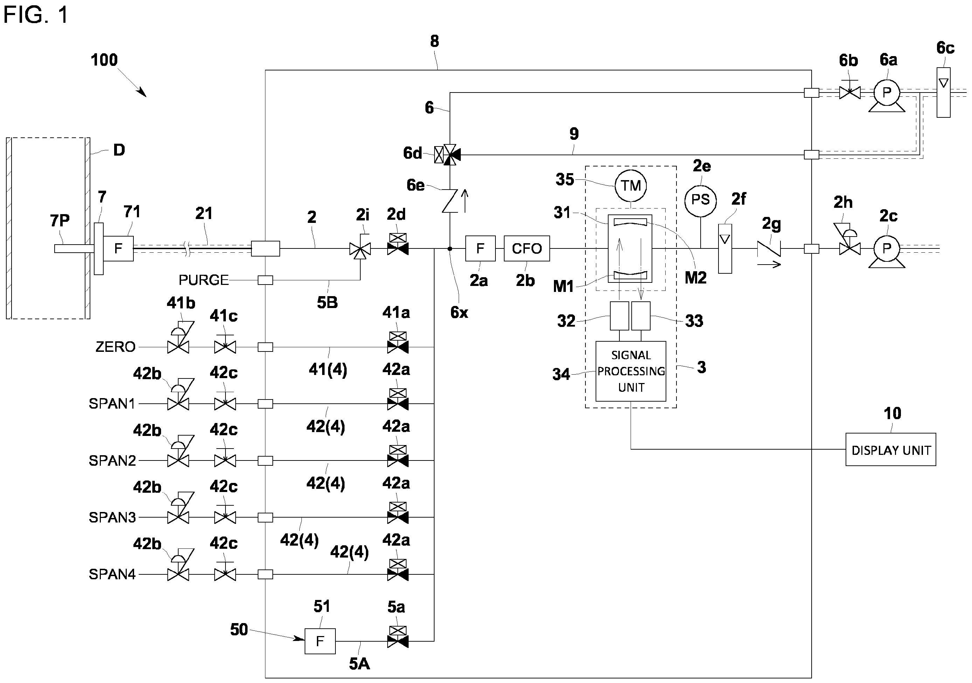

Concretely, as shown in Fig. 1, the gas continuous analysis system 100 comprises a main flow channel 2 through which the sample gas containing an adsorbable component flows, an analyzer 3 that analyzes the adsorbable component in the sample gas flowing in the main flow channel 2, a calibration gas flow channel 4 that is a standard gas flow channel supplying a calibration gas which is a standard gas to the analyzer 3, and a purge gas flow channel (first purge gas flow channel) 5A that supplies a purge gas (first purge gas) containing moisture to a flow channel through which the sample gas of the analyzer 3 flows and a second purge gas flow channel 5B that is connected to an upstream side of the analyzer 3 in the main flow channel 2 and that supplies a second purge gas from a connection point between the second purge gas flow channel 5B and the main flow channel 2 to the upstream side in the main flow channel 2, an overflow flow channel 6 that discharges a part of the sample gas flowing in the main flow channel 2 at the upstream side of the analyzer 3.

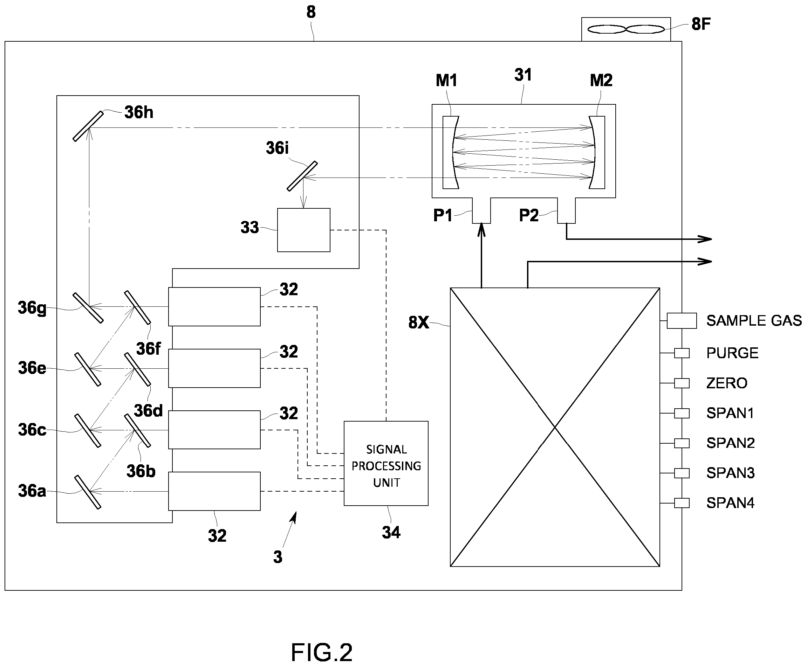

A sampling unit 7 for sampling the gas flowing in, for example, a flue is provided at one end part of the main flow channel 2. This sampling unit 7 has a sampling probe 7P that is inserted into the flue formed by the duct (D). The gas is sampled from the sampling probe 7P. The sampling unit 7 has a dust collection filter 71. The main flow channel 2 is made of a hot hose 21 from the sampling unit 7 to a housing 8 that houses the analyzer 3. If the hot hose 21 is made of Teflon, it is possible to prevent adsorption of adsorbable components. The code 8F in Fig. 2 is a ventilation unit such as a fan provided on a wall of the housing 8.

Concretely, the main flow channel 2 has a dust collection filter 2a, a throttle unit 2b such as an orifice, the analyzer 3 and a main suction pump 2c in this order from the upstream side in the downstream side of the hot hose 21. In addition, a solenoid switch valve 2d is provided in the upstream side of the dust collection filter 2a in the main flow channel 2. In addition, a pressure sensor 2e, a flow sensor 2f, a check valve 2g or a pressure regulating valve 2h may be provided between the analyzer 3 and the main suction pump 2c in the main flow channel 2. In this embodiment, the pressure regurating valve 2h and the main suction pump 2c are provided outside the housing 8. Furthermore, the piping in the downstream side of the main suction pump 2c in the main flow channel 2 may be heated to prevent moisture from condensing. In this embodiment, the heating temperature can be set according to the moisture concentration in the sample gas. If the moisture concentration in the sample gas is, for example, 6 vol%, it can be considered that the heating temperature is, for example, 40°C. In addition, a dehumidifier or a buffer tank may be provided in the downstream side of the main suction pump 2c to remove the moisture in the sample gas. The upstream side of the main suction pump 2c is depressurized and does not need to be heated, however, it may be heated or a dehumidifier or a buffer tank may be provided.

The analyzer 3 continuously analyzes the concentration of HF, HCl or NH₃ as the adsorbable component in the sample gas, using, for example, an IRLAM detector based on the infrared laser absorption modulation method (refer to the patent document 6886507).

Concretely, as shown in Fig. 1 and Fig. 2, the analyzer 3 comprises a measurement cell 31 into which the sample gas is introduced, a semiconductor laser 32 that irradiates a laser beam into the measurement cell 31, a photodetector 33 that receives the laser beam passing through the measurement cell 31, and a signal processing unit 34 that receives an output signal of the photodetector 33 and that calculates a concentration of the measured component based on a value of the output signal. The measurement cell 31 has an introduction port P1 for introducing the sample gas and a discharge port P2 for discharging the sample gas, and a pair of reflecting mirrors M1 and M2 are provided inside of the measurement cell 31 and is a so-called Herriott cell that multiply reflects the laser light. In addition, the measurement cell 31 is temperature-controlled to a predetermined temperature based on the temperature detected by a temperature sensor 35. Furthermore, the concentration of the measured component obtained by the signal processing unit 34 is displayed on a display unit 10, such as a display.

In this embodiment, a quantum cascade laser, an interband cascade laser, or other semiconductor laser can be used as the semiconductor laser 32.

The quantum cascade laser is a semiconductor laser that uses an intersubband transition with a multiple quantum well structure and that emits a laser beam with a specific wavelength in a wavelength range of about 4 µm to about 20 µm. In addition, the interband cascade laser is a semiconductor laser that uses an interband transition of a multi-stage PN junction with a quantum well structure and that emits a laser beam with a specific wavelength in a wavelength range of about 3 µm to about 5 µm. Furthermore, other semiconductor laser is a semiconductor laser that uses an interband transition of a single PN junction with a quantum well structure and that is capable of oscillating an ultraviolet light, a visible light, and a near-infrared light mainly in the 0.3 µm to 3 µm range.

Furthermore, the semiconductor laser 32 may comprise a plurality of semiconductor lasers 32 as shown in Fig. 2. In this case, a plurality of the semiconductor lasers 32 can be configured to emit laser beams of mutually different wavelength according to the measurement component. In this embodiment, a plurality of the semiconductor lasers 32 may be configured to emit the laser beams at different timings from each other, or at least two or more can emit the laser beam simultaneously. In addition, a plurality of the semiconductor lasers 32 may have a plurality of reflecting mirrors 36a to 36h for introducing the laser beam emitted from each of a plurality of the semiconductor lasers 32 into the measurement cell 31. In addition, the laser beam emitted through the measurement cell 31 is reflected by the reflecting mirror 36i and detected by the photodetector 33. The code 8X in Fig. 2 indicates a piping system (the main flow channel 2, the calibration gas flow channel 4, the first purge gas flow channel 5A, the second purge gas flow channel 5B or the overflow flow channel 6 or the like) provided in the housing 8 and used to introduce the sample gas, the purge gas or the calibration gas into the measurement cell 31 in an omitted manner.

The calibration gas flow channel 4 is connected in an upstream side of the analyzer 3 in the main flow channel 2 and supplies the calibration gas to the main flow channel 2. The calibration gas flow channel 4 in accordance with this embodiment is connected in the upstream side of the dust collection filter 2a in the main flow channel 2, concretely between the solenoid valve 2d and the dust collection filter 2a.

Concretely, the calibration gas flow channel 4 has a zero gas flow channel 41 that supplies the zero gas such as N₂ gas to the analyzer 3 and a span gas flow channel 42 that supplies a span gas of known concentration to the analyzer 3. In this embodiment, the zero gas flow channel 41 and the span gas flow channel 42 merge with each other and are connected to the main flow channel 2. In addition, the span gas flow channel 42 may be provided in plurality so that various span gases can be supplied ("SPAN1 to SPAN4" in Fig. 1). In this embodiment, each of the calibration gas flow channels 41 and 42 is provided with solenoid open/close valves 41a and 42a to switch the supply of each of the calibration gases. In addition, each of the calibration gas flow channels 41 and 42 may be provided with a pressure adjustment valves 41b and 42b and needle valves 41c and 42c for flow adjustment.

The first purge gas flow channel 5A is connected in the upstream side of the analyzer 3 in the main flow channel 2 and supplies the first purge gas containing moisture to the main flow channel 2 to supply the first purge gas containing moisture to the flow channel in which the sample gas of the analyzer 3 flows. The first purge gas flow channel 5A in this embodiment supplies atmosphere as the first purge gas containing moisture to the flow channel in which the sample gas of the analyzer 3 flows. For this purpose, a dust collection filter 51 is provided in an atmosphere introduction unit 50 of the first purge gas flow channel 5A. The first purge gas flow channel 5A is connected in an upstream side of the dust collection filter 2a in the main flow channel 2, concretely, between the solenoid switch valve 2d and the dust collection filter 2a. In addition, the first purge gas flow channel 5A joins the calibration gas flow channel 4 and is connected to the main flow channel 2. As a result, the first purge gas flow channel 5A supplies the atmosphere as the first purge gas also to the calibration gas flow channel 4. In this embodiment, the first purge gas flow channel 5A is provided with a solenoid open/close valve 5a to switch the supply of the first purge gas.

The second purge gas flow channel 5B is connected in the upstream side of the analyzer 3 in the main flow channel 2 and supplies the second purge gas to the main flow channel 2 to the upstream side of the connection point of the second purge gas flow channel 5B. The second purge gas of this embodiment is to discharge the exhaust gas stagnating in the main flow channel 2 to the outside from the sampling unit 7 by purging the exhaust gas by making the second purge gas flow back into the main flow channel 2. Although not shown in drawings, a second purge gas source (not shown in drawings) that pumps the second purge gas is connected to the upstream side of the second purge gas flow channel 5B. In this embodiment, dry air or nitrogen gas can be used as the second purge air. Concretely, the second purge air flow channel 5B is connected to the upstream side of the solenoid valve 2d in the main flow channel 2. In addition, a three-way valve 2i is provided at the connection point of the second purge gas flow channel 5B in the main flow channel 2. The three-way valve 2i may be a manual switching valve or a solenoid switching valve. The solenoid valve 2d in the main flow channel 2 may be a three-way valve, and the second purge gas flow channel 5B may be connected to this solenoid valve 2d.

The overflow channel 6 is connected to the upstream side of the analyzer 3 in the main flow channel 2 and discharges a part of the sample gas flowing in the main flow channel 2. In this embodiment, the overflow channel 6 is connected to the upstream side of the dust collection filter 2a in the main flow channel 2, concretely, between the solenoid open/close valve 2d and the dust collection filter 2a. In other words, only the dust collection filter 2a and the throttle unit 2b are provided between the connection point 6x of the overflow flow channel 6 and the analyzer 3, resulting in making a distance from the connection point 6x of the overflow flow channel 6 to the analyzer 3 as short as possible. In addition, the sub-suction pump 6a is provided in the overflow flow channel 6. A needle valve 6b for flow adjustment may be provided in an upstream side of the sub-suction pump 6a, and a flow sensor 6c may be provided in the downstream side of the sub-suction pump 6a. In this embodiment, the sub-suction pump 6a, the needle valve 6b, and the flow sensor 6c are arranged outside the housing 8. In addition, in this embodiment, the piping outside the housing 8 in the overflow flow channel 6 may be heated to prevent moisture from condensing. In this embodiment, the heating temperature can be set according to the moisture concentration in the sample gas. In case that the moisture concentration in the sample gas is, for example, 6 vol%, it is conceivable that the heating temperature is set, for example, at 40°C. In addition, the system can also be configured to remove the moisture in the sample gas by providing a dehumidifier or a buffer tank or the like in the piping outside the housing 8 in the overflow flow channel 6.

Furthermore, a bypass channel 9 that bypasses the sub-suction pump 6a is connected to the overflow flow channel 6. The three-way solenoid valve 6d is provided at the connection point in the upstream side of the overflow flow channel 6 and the bypass channel 9 to enable switching of the flow channels. In addition, a check valve 6e may be provided in the upstream side of the three-way solenoid valve 6d in the overflow flow channel 6. In this embodiment, the piping outside the housing 8 in the bypass channel 9 may be heated to prevent moisture from condensing. The heating temperature can be set according to the moisture concentration in the sample gas, and in case that the moisture concentration in the sample gas is, for example, 6 vol%, it is conceivable that the heating temperature may be, for example, 40°C. The system may be configured to remove the moisture in the sample gas by providing a dehumidifier or buffer tank or the like in the piping outside the housing 8 in the bypass channel 9.

The piping inside the housing 8 in the main flow channel 2, the overflow flow channel 6 or the bypass flow channel 9 may be heated to prevent moisture from condensing. Concretely, in the main flow channel 2, the piping connected to the hot hose 21 up to the orifice 2b may be heated. In this embodiment, the downstream side of the orifice 2b is depressurized by the main suction pump 2c and does not necessarily need to be heated. In addition, the system may be configured to partially heat the area prone to condensation depending on the measurement condition or the use condition.

In addition, since the gas continuous analysis system 100 of this embodiment continuously analyzes a corrosive gas such as hydrogen fluoride (HF) or hydrogen chloride (HCl) in the sample gas, the flow channels through which the sample gas flows may be made of a material that is resistant to a corrosive gas. For example, the piping that constitutes the main flow channel 2, the piping that constitutes the overflow flow channel 6, and/or the piping that constitutes the bypass flow channel 9 may be made of a Teflon pipe or a stainless steel pipe (for example, SUS316L). In case that the stainless steel pipe is used, it is conceivable to use the pipe with polished inner surfaces. For facilitating piping, it is desirable to use the Teflon pipe. The measurement cell 31 may be made of a material that is resistant to a corrosive gas. For example, it is conceivable that the measurement cell 31 is made of stainless steel (for example, SUS316L), and the reflecting mirrors M1 and M2 arranged inside the measurement cell 31 is made of stainless steel (for example, SUS316L) with gold plating on the reflecting surface. In addition to stainless steel, Hastelloy or Inconel, or the like may be used.

### < Operation of the gas continuous analysis system 100>

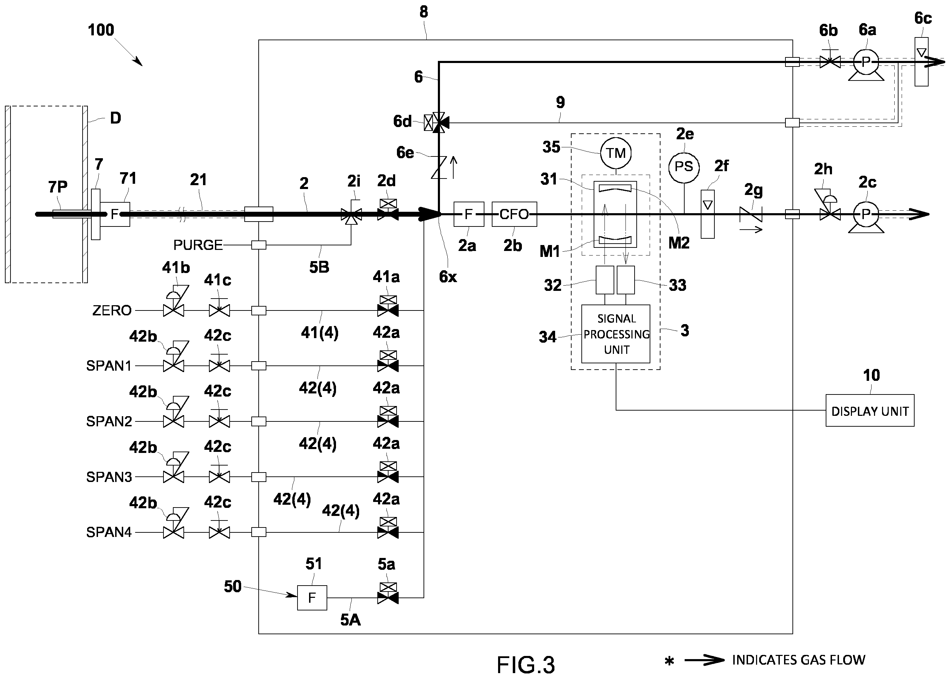

An operation of the gas continuous analysis system 100 of this embodiment will be described below with reference to Fig. 3 through Fig. 7. The operation of the gas continuous analysis system 100 implements the gas continuous analysis method.

### <Normal analysis

During a normal continuous analysis, as shown in Fig. 3, the sample gas of a sum of a flow rate of a suction flow rate of the main suction pump 2c provided in the main flow channel 2 and a suction flow rate of the sub suction pump 6a provided in the overflow flow channel 6 is sampled. A part of the sample gas flowing in the main flow channel 2 is then introduced into the analyzer 3 through the collecting filter 2a and the throttle unit 2b. Other sample gas is exhausted through the overflow channel 6. The sample gas sucked by the main suction pump 2c and introduced into the analyzer 3 through the throttle unit 2b is at a constant flow rate, and the inside of the measurement cell 31 is in a depressurized state. With this state kept, the concentration of HF or HCl, which is the adsorbable component is continuously analyzed by the analyzer 3.

### <Calibration (including purge)>

In the middle of the above-mentioned continuous analysis, a calibration process is periodically performed based on, for example, a predetermined schedule. Before this calibration process, a first purge process is performed.

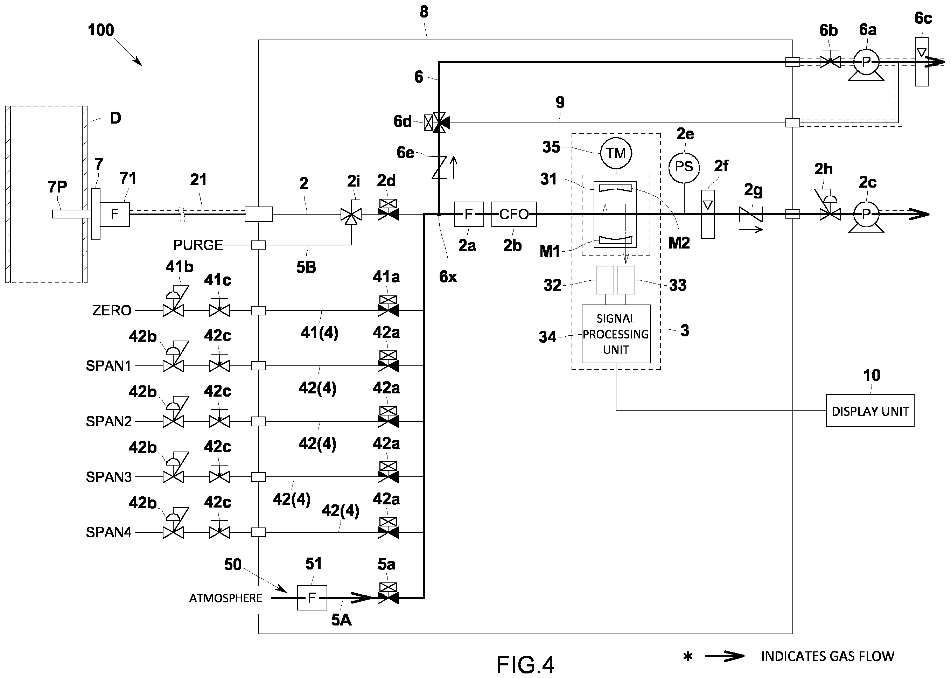

Concretely, as shown in Fig. 4, the first purge gas is supplied from the first purge gas flow channel 5A to the analyzer 3 to purge the flow channel through which the sample gas flows in the analyzer 3 and a part of the calibration gas flow channel 4. In this embodiment, a flow channel through which the sample gas flows is a flow channel from the sample gas introduction port (not shown in drawings) of the analyzer 3 to the measurement cell 31. In addition, a part of the calibration gas flow channel 4 is a part in the downstream side of the connection point where the first purge gas flow channel 5A joins the calibration gas flow channel 4. At this time, the solenoid open/close valve 2d of the main flow channel 2 is closed and the solenoid open/close valve 5a of the first purge gas flow channel 5A is opened. With this state kept, the atmosphere containing moisture is introduced from the atmosphere introduction unit 50 of the first purge gas flow channel 5A by the main suction pump 2c to introduce the atmosphere containing moisture into the measurement cell 31 of the analyzer 3. As a result of this, adsorbed components adsorbed on the inner surface of the piping from the connection point of the first purge gas flow channel 5A in the main flow channel 2 to the measurement cell 31, the inner surface of the measurement cell 31, and the reflecting mirrors M1 and M2 are separated and removed by water molecules.

Following the above-mentioned first purging process, the analyzer 3 is calibrated by supplying the calibration gas to the analyzer 3 from the calibration gas flow channel 4. In other words, the analyzer 3 is calibrated in a purged state.

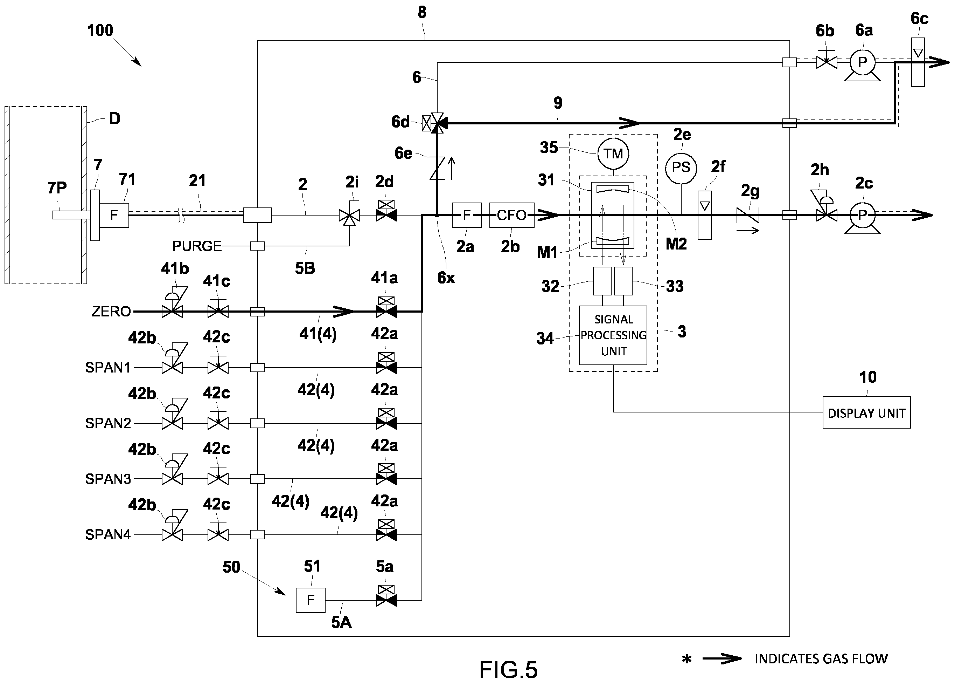

First, as shown in Fig. 5, zero calibration is performed by supplying the zero gas from the zero gas flow channel 41 to the analyzer 3. In this embodiment, the solenoid open/close valve 2d of the main flow channel 2 is closed and the solenoid open/close valve 41a of the zero gas flow channel 41 is opened. With this state kept, the zero gas is introduced into the measurement cell 31 of the analyzer 3 from the zero gas flow channel 41 by the main suction pump 2c. Then, the analyzer 3 is zero calibrated. Even if the calibration gas is supplied in a pressurized state from a cylinder, it is possible to minimize the pressure difference at the connection point 6x at a time when the sample gas flows into the main flow channel 2 (Fig. 3) or the first purge gas flows into the main flow channel 2 (Fig. 4) by opening the three-way solenoid valve 6d in the overflow flow channel 6 to the atmosphere.

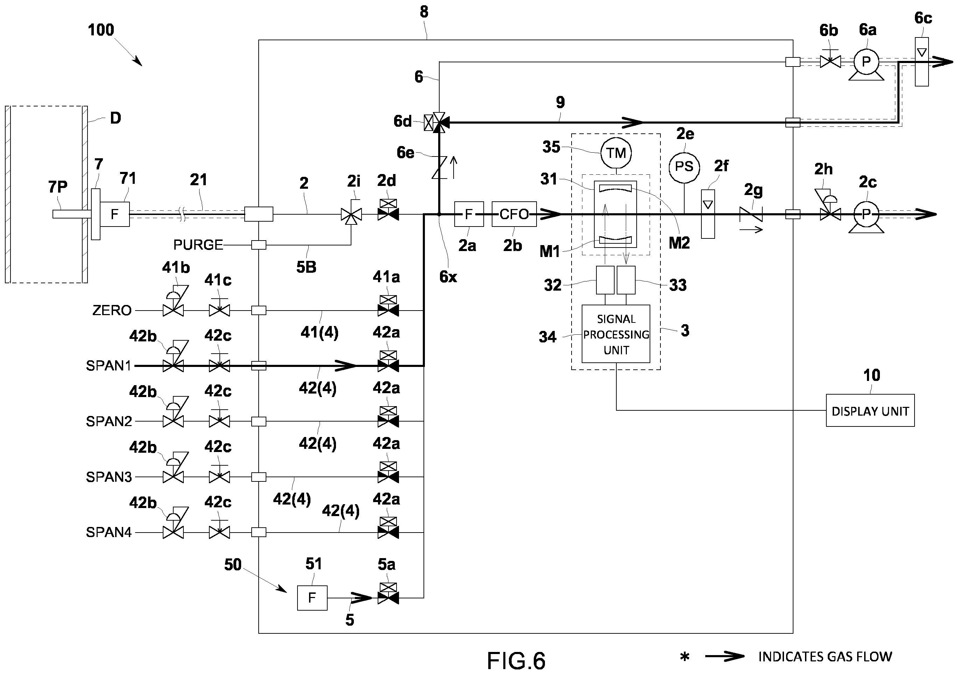

Next, as shown in Fig. 6, span calibration is performed by supplying the span gas from the span gas flow channel 42 to the analyzer 3. In this embodiment, the solenoid open/close valve 2d of the main flow channel 2 is closed and either one of the solenoid open/close valves 42a of the span gas flow channel 42 is opened. With this state kept, span gas is introduced from the span gas flow channel 42 to the measurement cell 31 of the analyzer 3 by the main suction pump 2c. Then, the analyzer 3 is span calibrated. Even if the calibration gas is supplied in a pressurized state from the cylinder, it is possible to minimize the pressure difference at the connection point 6x at a time when the sample gas flows into the main flow channel 2 (Fig. 3) or the first purge gas flows into the main flow channel 2 (Fig. 4) by opening the three-way solenoid valve 6d in the overflow flow channel 6 to the atmosphere.

After the series of the above-mentioned operations of the purge process, the zero calibration, and the span calibration are completed, continuous analysis is performed again. Concretely, when the series of the above-mentioned operations are completed, the solenoid open/close valve 2d in the main flow channel 2 is opened to resume introduction of the sample gas into the analyzer 3, and the three-way solenoid valve 6d in the overflow flow channel 6 shuts off the bypass channel 9 to resume exhaust of the sample gas by the overflow flow channel 6 (refer to Fig. 3).

### <At the end of continuous analysis (before or at a time of system shutdown)>

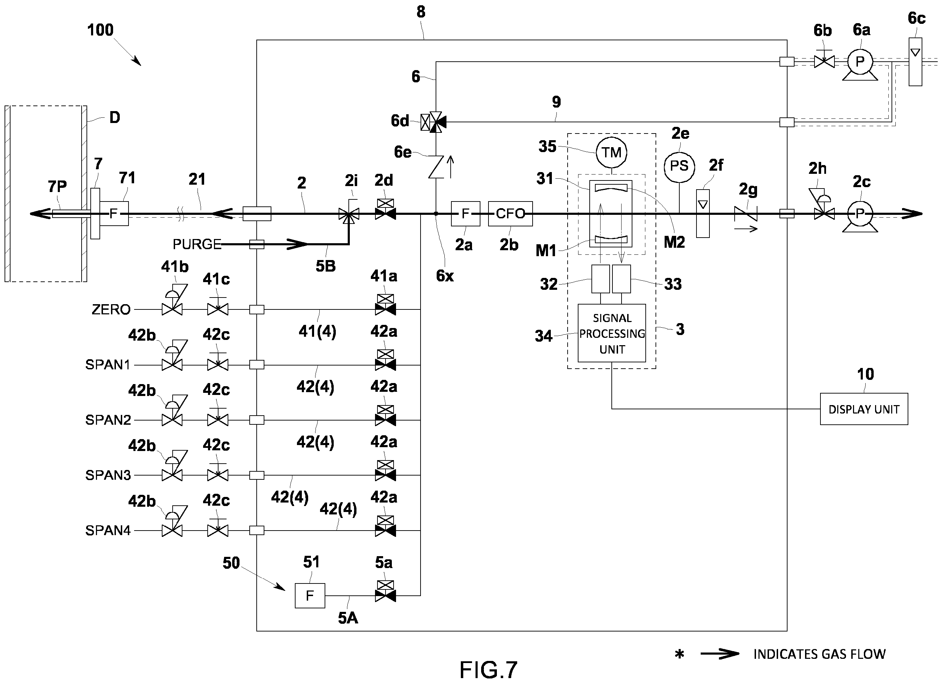

When the above continuous analysis is completed, the three-way valve 2i is switched to supply the second purge gas from the second purge gas flow channel 5B before or at a time of system shutdown, as shown in Fig. 7. In this embodiment, three ports of the three-way valve 2i are in a state where the port connected to the downstream side of the main flow channel 2 is closed and the port connected to the upstream side of the main flow channel 2 and the port connected to the second purge gas flow channel 5B are connected. In the abovementioned <normal analysis> and <calibration (including purge)>, the three ports of the three-way valve 2i are in a state where the port connected to the second purge gas flow channel 5B is closed and the port connected to the upstream side of main flow channel 2 is connected to the downstream side of main flow channel 2.

The second purge gas is allowed to flow back into the main flow channel 2 through the three-way valve 2i. As a result of this, the exhaust gas stagnating in the main flow channel 2 is purged and discharged from the sampling unit 7 to the outside (second purge process). With this state kept, the gas continuous analysis system is stopped. In the second purge process where the second purge gas is supplied from the second purge gas flow channel 5B to the upstream side of the main flow channel 2, the first purge gas may be supplied to the downstream side of the main flow channel 2 to purge the analyzer 3, similar to the above-mentioned <calibration (including purge) >.

### <Effect of this embodiment>

In accordance with the gas continuous analysis system 100 having the above configuration, since the flow channel through which the sample gas of the analyzer 3 flows is purged using the first purge gas containing moisture before calibrating the analyzer 3, it is possible to shorten the time until the indicated value of the analyzer 3 becomes stable during calibration. In this embodiment, since the first purge gas containing moisture is used, the adsorbed component adsorbed on the inner surface of the measurement cell 31 of analyzer 3 are easily desorbed by water molecules. As a result of this, the calibration time between consecutive analyses can be shortened, and the interruption time in the continuous analysis of the sample gas can be reduced.

### <Other embodiments>

For example, in the gas analysis system 100 of the above-mentioned embodiment, during normal continuous analysis, the sample gas is suctioned by the sub-suction pump 6a and overflows through the overflow channel 6, and during calibration, the calibration gas is pressurized and supplied from the calibration gas cylinder in a state wherein the bypass flow channel 9 is opened to the atmosphere. In other words, in the above-mentioned embodiment, the pressure at the connection point 6x of the overflow flow channel 6 during continuous analysis is not the same as that during calibration, and the internal pressure of the measurement cell 31 differs due to this effect, resulting in changes in the indicated value of the analyzer 3.

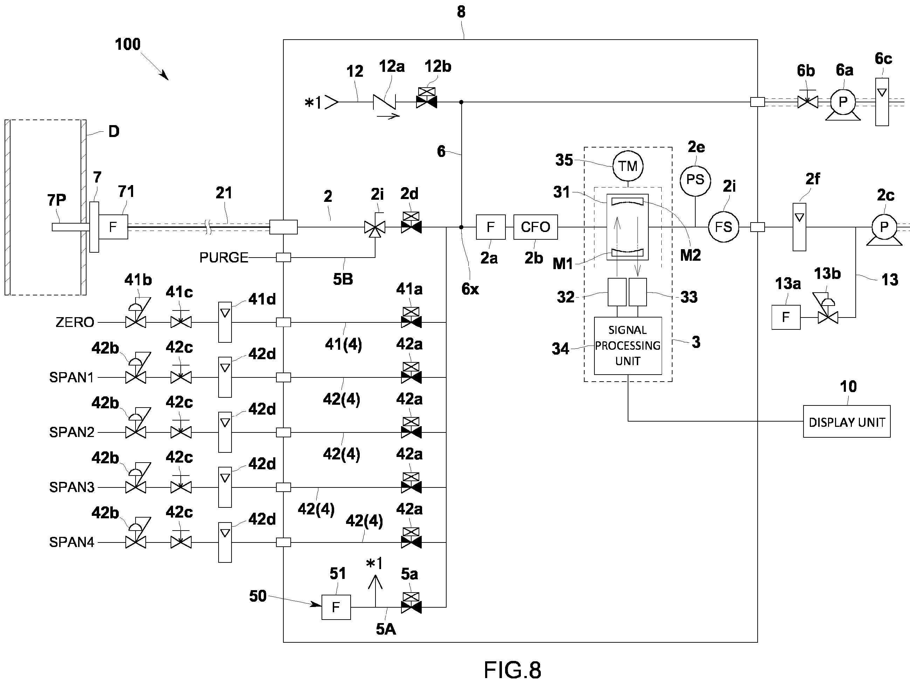

Therefore, as shown in Fig. 8, it is preferable to eliminate the bypass flow channel 9 and to suck the sample gas through the overflow flow channel 6 by means of the sub-suction pump 6a during both the normal continuous analysis and calibration. The overflow channel 6 is connected to a compensation gas introduction channel 12 that introduces a compensation gas such as air. The compensation gas introduction channel 12 is provided with a check valve 12a and a solenoid open/close valve 12b. In addition, *1 in Fig. 8 indicates that the compensation gas introduction channel 12 is branched from the first purge gas flow channel 5A, and the atmosphere that has passed through the dust collection filter 51 is introduced into the compensation gas introduction channel 12. The compensation gas introduction channel 12 may be provided independently of the first purge gas flow channel 5A.

In case that the calibration gas is pressurized and supplied to the measurement cell 31 from the calibration gas cylinder (during calibration), the solenoid open/close valve 12b is opened so that the compensation gas is supplied from the compensation gas flow channel 12 to the overflow flow channel 6. This allows the compensation gas to compensate for the shortage of the calibration gas flow rate relative to the suction flow rate of the sub-suction pump 6a during calibration. As a result of this, it is possible to reduce the pressure difference at the connection point 6x between at a time of continuous analysis and at a time of calibration.

In addition, it is necessary to flow a predetermined flow rate of gas of, for example, 1.5 L/min in the main flow channel 2, and if no more calibration gas is supplied from the calibration gas cylinder during calibration, the compensation gas will flow back into the main flow channel 2 through the compensation gas flow channel 12. Therefore, it is preferable to provide calibration gas flow sensors 41d and 42d in each of the calibration gas flow channels 4 (41, 42). In accordance with this configuration, it is possible to accurately measure the calibration gas supplied to the main flow channel 2 and to prevent the compensation gas from flowing back into the main flow channel 2 through the compensation gas flow channel 12.

Furthermore, the load of the main suction pump 2c fluctuates between at a time of a normal continuous measurement and at a time of calibration. As a result of this, in case that calibration is performed at a time of continuous measurement and then continuous measurement is performed again, the internal pressure of the measurement cell during continuous measurement after calibration changes from the internal pressure of the measurement cell during continuous measurement before calibration, resulting in a problem of poor reproducibility.

Therefore, as shown in Fig. 8, a pressure adjustment channel 13 is connected between the measurement cell 31 and the main suction pump 2c in the main flow channel 2 to reduce a load fluctuation of the main suction pump 2c during continuous measurement and calibration. In this embodiment, the pressure adjustment channel 13 suppresses the load fluctuation of the main suction pump 2c by introducing the atmosphere into the main flow channel 2. The dust collection filter 13a and the pressure adjustment valve 13b are provided in the pressure adjustment channel 13. In accordance with this configuration, it is possible to reduce the load fluctuation of the main suction pump 2c during continuous measurement and calibration. In addition, in order to measure the flow rate of the gas flowing through the measurement cell 31, a flow sensor 2s is provided between the measurement cell 31 and the pressure adjustment flow channel 13.

In the above-mentioned embodiment, the zero calibration and the span calibration are performed after the first purge process, however, the first purge process and the zero calibration may be performed simultaneously. In this case, the gas continuous analysis system 100 supplies the first purge gas from the first purge gas flow channel 5A to the analyzer 3 to purge the analyzer 3 and to zero-calibrate the analyzer 3 in the middle of continuous analysis and continues to supply the span gas from the calibration gas flow channel 4 to the analyzer 3 to span-calibrate the analyzer 3.

In addition, in the above-mentioned embodiment, the configuration has the overflow channel 6, however, it can also be configured without an overflow channel 6.

In the above-mentioned embodiment, the configuration has the second purge gas flow channel 5B, however, it can also be configured without a second purge gas flow channel 5B.

In the above-mentioned embodiment, the standard gas flow channel 4 supplies the calibration gas as being a standard gas for calibrating the analyzer 3, however, it may also supply a validation gas as being a standard gas for validating the analyzer 3. The validation gas is a gas used to check the performance of the analyzer 3. By supplying the validation gas to the analyzer 3, it is possible to verify the performance of the analyzer 3.

Furthermore, the analyzer 3 in the above-mentioned embodiment uses the IRLAM detector, however, it can also be a detector using an infrared absorption method, such as a detector using non-dispersive infrared absorption (NDIR) or a detector using Fourier transform infrared spectroscopy (FTIR).

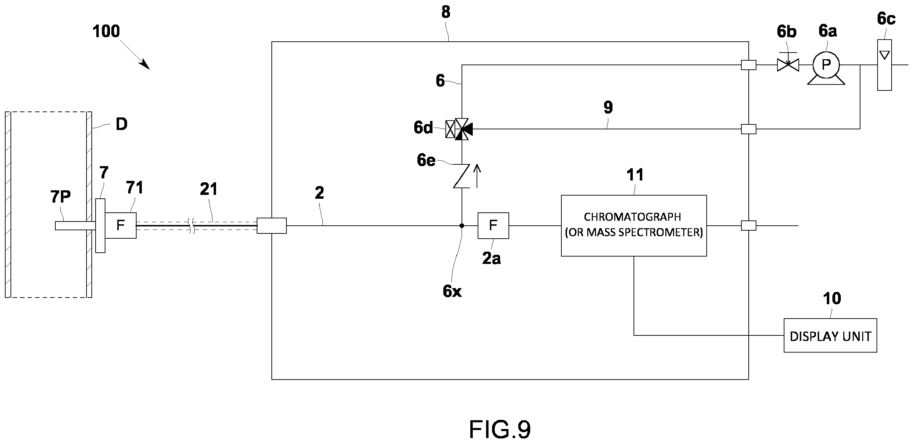

In addition, as shown in Fig. 9, the gas continuous analysis system of this invention comprises a sampling unit 7 for sampling a gas containing an adsorbable component flowing in a duct, a main flow channel 2 through which the sample gas sampled by the sampling unit 7 flows, a chromatograph or a mass spectrometer 11 for analyzing the adsorbable component in the sample gas flowing in the main flow channel 2, and an overflow flow channel 6 for discharging a part of the sample gas flowing in the main flow channel 2 in the upstream side of the chromatograph or the mass spectrometer 11. The chromatograph can be an ion chromatograph, a gas chromatograph, or the like. In Fig. 8, the configuration with the same codes as in the above-mentioned embodiment is the same as that in the above-mentioned embodiment. In accordance with this configuration, the sample gas introduced into the main flow channel 2 can be made to flow at a higher rate by the overflow flow channel 6. As a result of this, it is possible to make it difficult for the adsorbable component to be adsorbed on the inner surface of the piping that constitutes the main flow channel 2, and it is possible to improve the accuracy of analysis of a subtle amount of the adsorbable component by the chromatograph or the mass spectrometer 11.

The present claimed invention may be variously modifying or combining the embodiment without departing from a spirit of the invention.

### Possible applications in industry

In accordance with the present claimed invention, it is possible to shorten the time required to conduct calibration or validation on the analyzer that analyzes the sample gas containing an adsorbable component.

### Explanation of codes

- 100: infrared gas analyzer
- 2: main flow channel
- 3: analyzer
- 4: calibration gas flow channel (standard gas flow channel)
- 41: zero gas flow channel
- 42: span gas flow channel
- 5A: purge gas flow channel (first purge gas flow channel)
- 5B: second purge gas flow channel
- 6: overflow flow channel
- 2b: throttle unit
- 2c: main suction pump
- 6a: sub-suction pump

## Claims

1. A gas continuous analysis system, comprising
a main flow channel through which a sample gas containing an adsorbable component flows,
an analyzer that analyzes the adsorbable component in the sample gas flowing in the main flow channel,
a standard gas flow channel that supplies a standard gas for calibrating or validating the analyzer, and
a purge gas flow channel that supplies a purge gas containing moisture to a flow channel through which the sample gas of the analyzer passes, wherein
the sample gas is introduced into the main flow channel and the sample gas is continuously analyzed by the analyzer, and in the middle of the continuous analysis, the purge gas is supplied to the analyzer from the purge gas flow channel to purge the flow channel through which the sample gas of the analyzer passes, and successively a standard gas is supplied to the analyzer from the standard gas flow channel to calibrate or validate the analyzer.

2. The gas continuous analysis system described in claim 1, wherein
the purge gas flow channel supplies atmosphere to the flow channel through which the sample gas of the analyzer gas passes.

3. The gas continuous analysis system described in claim 1 or claim 2, wherein
the analyzer analyzes HF, HCl or NH₃ as the adsorbable component.

4. The gas continuous analysis system described in either one of claim 1 through claim 3, wherein
the standard gas flow channel has a zero gas flow channel that supplies a zero gas to the analyzer, and a span gas flow channel that supplies a span gas to the analyzer, wherein
the gas continuous analysis system supplies the purge gas to the analyzer from the purge gas flow channel in the middle of continuous analysis to purge the flow channel through which the sample gas of the analyzer passes, successively supplies the zero gas from the zero gas flow channel to the analyzer to zero-calibrate the analyzer, and supplies the span gas to the analyzer from the span gas flow channel to span-calibrate the analyzer.

5. The gas continuous analysis system described in either one of claim 1 through claim 3, wherein
the standard gas flow channel supplies the span gas to the analyzer, and
the gas continuous analysis system supplies the purge gas to the analyzer from the purge gas flow channel to purge the flow channel through which the sample gas of the analyzer passes and to zero-calibrate the analyzer, and successively supplies the span gas to the analyzer from the standard gas flow channel to span-calibrate the analyzer.

6. The gas continuous analysis system described in either one of claim 1 through claim 5, further comprising
a second purge gas flow channel that is connected to an upstream side of the analyzer in the main flow channel and that supplies a second purge gas from a connection point of the second purge gas flow channel to the main flow channel toward the upstream side of the main flow channel, wherein
after the continuous analysis is completed, the gas continuous analysis system supplies the second purge gas from the second purge gas flow channel to purge the upstream side of the connection point in the second purge gas flow channel.

7. The gas continuous analysis system described in either one of claim 1 through claim 6, further comprising
an overflow flow channel that discharges a part of the sample gas flowing in the main flow channel in the upstream side of the analyzer.

8. The gas continuous analysis system described in claim 7, wherein
a throttle unit is provided between a connection point of the overflow flow channel to the main flow channel and the analyzer in the main flow channel, and a main suction pump is provided in the downstream side of the analyzer in the main flow channel, and
a sub-suction pump is provided in the overflow flow channel.

9. A gas continuous analysis system comprising
a sampling unit that samples a gas containing an adsorbable component flowing in a duct,
a main flow channel through which a sample gas sampled by the sampling unit flows,
a chromatograph or a mass spectrometer that analyzes an adsorbable component in the sample gas flowing in the main flow channel, and
an overflow channel that discharges a part of the sample gas flowing in the main flow channel in an upstream side of the chromatograph or the mass spectrometer.

10. A gas continuous analysis method using a measurement system that comprises a main flow channel through which a sample gas containing an adsorbable component flows, an analyzer that analyzes the adsorbable component in the sample gas flowing in the main flow channel, a standard gas flow channel that supplies a standard gas for calibration or validation to the analyzer, and a purge gas flow channel that supplies a purge gas containing moisture to the flow channel through which the sample gas of the analyzer flows, wherein
the sample gas is introduced into the main flow channel and the sample gas is continuously analyzed by the analyzer, the purge gas is supplied to the analyzer from the purge gas flow channel in the middle of continuous analysis to purge the flow channel through which the sample gas of the analyzer passes, and successively the standard gas is supplied to the analyzer from the standard gas flow channel to calibrate or validate the analyzer.
